# EUROPÄISCHE PATENTSCHRIFT

(11) **EP 1 093 385 B1**
(45) Veröffentlichungstag und Bekanntmachung des Hinweises auf die Patenterteilung: **20.12.2006**
(21) Anmeldenummer: 99939771.4
(22) Anmeldetag: 26.05.1999
(51) Int. Cl.: A61L 27/40, A61B 17/80, A61B 17/86

(54) **VERBUNDWERKSTOFF AUS POLYMER- ODER KERAMIKMATERIALIEN SOWIE BAUTEIL AUS EINEM SOLCHEN VERBUNDWERKSTOFF**
COMPOSITE OF POLYMER OR CERAMIC MATERIALS AND COMPONENT MADE OF SUCH A COMPOSITE
MATERIAU COMPOSITE CONSTITUE DE MATERIAUX POLYMERES ET CERAMIQUES, ET PIECE REALISEE A PARTIR DUDIT MATERIAU COMPOSITE

(30) Priorität: 27.05.1998 DE 19823737
(43) Veröffentlichungstag der Anmeldung: 25.04.2001
(73) Patentinhaber: Sepitec Foundation, 9490 Vaduz (LI)
(72) Erfinder: MAGERL, Fritz, CH-9011 St. Gallen (CH); WINTERMANTEL, Erich, CH-5442 Fislisbach (CH); MAYER, Jörg, CH-5702 Niederlenz (CH); TOGNINI, Roger Roland, CH-9443 Widnau (CH); PETER, Thomas, Andreas, CH-8606 Nänikon (CH); SPIRIG, Walter, CH-9428 Platz-Walzenhausen (CH)
(74) Vertreter: Hofmann, Ralf U.
(86) Internationale Anmeldenummer: PCT/EP1999/003618
(87) Internationale Veröffentlichungsnummer: WO 1999/061081

(56) Entgegenhaltungen:
- EP-A- 0 551 574
- EP-A- 0 572 751
- GB-A- 2 203 342

## Beschreibung

Die Erfindung betrifft einen Verbundwerkstoff aus Polymer- oder Keramikmaterial mit einem Gehalt an integrierten Verstärkungselementen in Form von Fasern oder faserartigen Teilen für auf Zug, Biegung, Scherung, Druck und/oder Torsion beanspruchte Bauteile für den Einsatz bei Implantaten, Osteosyntheseplatten, Endoprotheseteilen, Verschraubungselementen oder bei chirurgischen Instrumenten.

Verbundwerkstoffe der verschiedensten Zusammensetzung erlangen gerade im Bereich der Chirurgie als Implantate ständig eine größere Akzeptanz. Schon bei der Fertigung können vorteilhafte Erkenntnisse einfließen, zumal die Schrumpfung bei der Polymerisation gegenüber reinen Kunststoffen verbessert wird. Auch die mechanischen Festigkeitswerte, wie z.B. die Druckfestigkeit, die Biegesteifigkeit und der Elastizitätsmodul werden verbessert Zudem kann der thermische Ausdehnungskoeffizient gegenüber dem reinen-Kunststoff vermindert werden.

So zeigt die EP-A-0 551 574 ein Implantat aus einem mehrschichtigen Verbundwerkstoff zur Erzielung hoher Festigkeit, hergestellt aus Thermoplasten wie z.B. Polyarylketonen, welcher im Inneren geflochtene metallische Fasern enthält, so daß das Implantat radiologisch einfach erkennbar ist. Die metallischen Fasern bewirken keine Erhöhung der Festigkeit der nichtmetallischen Materials ansich.

Die EP-A-0 572 751 beschreibt eine Endoprothese aus kompaktem, thermoplastischem Verbundwerkstoff aus Thermoplasten wie z.B. Polyarytetherkstonen und Endlosfasern (mit Bereichen unterschiedlicher Faserorientierung), wobei die Endoprothese ein Metallgitter und zusätzliche Röntgen marken enthält zur Sichtbarmachung bei Röntgenaufnahmen.

Die GB-A-2 203 342, die der nächste Stand der Technik ist, aber keinen Verbundwerkstoff offenbart, beschreibt ein Implantat z.B. aus gewobenem Polyester, welches zur Detektier barkeit nicht aber zur Verbesserung der Festigkeit, ein Metall (Au, Pt, Ti, Pd) als Draht oder Gitter enthält. Vielfach wird bei aus solchen Verbundwerkstoffen gefertigten Bauteilen als nachteilig empfunden, daß bei Röntgenuntersuchungen die eingesetzten Implantate, wie eben Osteosyntheseplatten, Knochenschrauben od.dgl. nicht erkannt werden können. Es werden daher vielfach noch gerade aus diesem Grund Implantate eingesetzt, die aus Metall bestehen oder Teile aus Metall enthalten.

In der Dentaltechnik ist es bekannt, einen röntgenopaken Werkstoff einzusetzen, der ein entsprechendes Zahnfüllmaterial bei Röntgenaufnahmen sichtbar machen soll, doch kann ein solcher Werkstoff nicht für Implantate eingesetzt werden, welche eine entsprechende Festigkeit aufweisen sollen und die einen entsprechend hohen Anteil von die Festigkeit erhöhenden Fasern haben. Wenn dann noch ein röntgenopaker Füllstoff zusätzlich in das Matnxrnaterial eingesetzt würde, wäre nicht mehr gewährleistet, daß die eingesetzten Fasern noch richtig eingebettet sind. Eine wesentliche Verminderung der Festigkeit eines solchen Bauteiles wäre die Folge. Es ist in einem faserverstärkten Verbundmaterial nicht einfach möglich, neben den Fasern auch noch andere Füllstoffe einzubringen.

Die vorliegende Erfindung hat sich daher zur Aufgabe gestellt, einen Verbundwerkstoff der eingangs genannten Art zu schaffen, bei dessen Einsatz gleichbleibende oder teils sogar verbesserte Festigkeitseigenschaften der daraus hergestellten Bauteile erzielt werden können, wobei außerdem eine gute Sichtbarkeit bei der Röntgendiagnostik ermöglicht werden soll.

Erfindungsgemäß gelingt dies dadurch, daß in dem Polymer- oder Keramikmaterial zumindest ein geringer Anteil der Verstärkungselemente durch Verstärkungselemente ersetzt ist, die aus einem Material bestehen, dessen Röntgenabsorption höher als die des Materials der übrigen Verstärkungselemente ist.

Diese Maßnahme bringt trotz Anordnung zusätzlicher Verstärkungselemente wie z.B. Fasern oder aber auch durch Austausch oder den teilweisen Austausch mit den sonst schon vorgesehenen Fasern eine Festigkeit des Verbundwerkstoffes, die gleich gut oder sogar noch besser ist als in der bisherigen Ausgestaltung. Solche Fasern tragen neben dem Effekt der möglichen Röntgendiagnostik bei den Implantaten zu einer entsprechenden Festigkeit bei. Solche Fasern aus einem Material mit höherer Röntgenabsorption ermöglichen die Röntgensichtbarkeit, ohne in der Regel andere bildgebende Verfahren wie CT, NMR, MRI od.dgl. zu stören. Auch bei Bestrahlungstherapien sind solche Fasern nicht störend, da sie keine relevante Schattenwirkung hervorrufen. Der wesentlichste Vorteil liegt aber eben darin, daß mit den Fasern oder faserartigen Teilen mit höherer Röntgenabsorption eher eine Festigkeitserhöhung der daraus hergestellten Implantate erzielt wird. Im Gegensatz dazu wird durch andere Füllstoffe oder andere röntgenopake Mischungen, z.B. partikuläre Metalloxide, die Festigkeit vermindert.

Weiter wird bei dem Verbundwerkstoff vorgeschlagen, daß dieser aus einem Polymer- oder Keramikmaterial mit einem hohen Anteil mit an Endlos-, Lang- oder Kurzfasern besteht, wobei zumindest ein geringer Anteil von Fasern oder faserartiger Teile aus einem Material höherer Röntgenabsorption vorhanden ist. Trotz eines sehr hohen Anteils an Endlosfasern kann der Volumenanteil des restlichen Materials beibehalten werden, und durch den alleinigen Austausch von sonst vorhandenen Fasern durch Fasern aus einem Material mit höherer Röntgenabsorption können die bestehenden Festigkeitseigenschaften erhalten oder sogar noch erhöht werden.

Eine vorteilhafte Ausführung sieht vor, daß der Verbundwerkstoff als Profilstangenmaterial aus Thermoplasten mit Kohlenstofffasern und Fasern aus einem Material mit höherer Röntgenabsorption besteht. Der Verbundwerkstoff ist in einem Warmumformverfahren in eine für das endgültige Bauteil erforderliche Form pressbar oder gepresst. Trotz der besonderen Zusammensetzung mit Fasern verschiedener Materialen kann die gute Warmumformbarkeit erhalten bleiben, so daß auch mit einem solcherart verbesserten Verbundwerkstoff eine optimale Fertigung auch relativ komplizierter Bauteile möglich ist

Bei einem Ausführungsbeispiel wird vorgeschlagen, daß der Verbundwerkstoff aus mit Kohlenstofffasern verstärktem PAEK (Poly-Aryl-Ether-Keton) und einem Anteil von Fasern aus einem Material mit höherer Röntgenabsorption gebildet ist. Es ist somit ein Werkstoff mit besonderer Verträglichkeit, mit großer Festigkeit und auch mit der für die Röntgendiagnostik notwendigen Sichtbarkeit geschaffen worden.

Optimale Festigkeitswerte können erzielt werden, wenn die Kohlenstofffasern und die Fasern aus einem Material mit höherer Röntgenabsorption als Endlosfasern und/oder Fasern mit einer Länge von mehr als 3 mm ausgebildet sind.

Damit eine Kraftübertragung zwischen den Fasern und dem anderen Werkstoff des Verbundwerkstoffes möglich ist, damit also auch bei großer Volumendichte der Fasern eine optimale Festigkeit gewährleistet ist, ist vorgesehen, daß die eingesetzten Fasern oder faserartigen Teile sowohl im Rohling als auch im fertigen Bauteil oberflächendeckend vom Matrixmaterial umschlossen sind.

An sich wären Fasern aus Stahl ebenfalls als röntgenopake Mittel einsetzbar, jedoch ergeben sich dann wieder andere Probleme bei Implantaten, wie z.B. Artefakte im MRI, NMR od.dgl. Es ist daher vorteilhaft, wenn die Fasern oder faserartigen Teile aus einem Material mit höherer Röntgenabsorption aus einem nichtmagnetischen Werkstoff gebildet sind.

Als besonders vorteilhaft wird daher angesehen, daß die Fasern oder faserartigen Teile mit höherer Röntgenabsorption aus Tantal, Wolfram, Gold, Platin od.dgl., also einem Metall oder aus Metalloxiden mit hohem Attenuationskoeffizienten, bestehen.

Bei einem aus dem erfindungsgemäßen Verbundwerkstoff hergestellten Bauteil sind in Anpassung an die Form und den Einsatz des Bauteiles ein vorherbestimmbarer Verlauf und eine vorherbestimmbare Menge und Ausrichtung von Verstärkungselementen in Form von Fasern oder faserartigen Teilen aus einem Material mit höherer Röntgenabsorption vorgesehen. Es ist damit auch die Sichtbarkeit des Bauteiles, also eines Implantates, abstufbar. Je nachdem, in welchen Abschnitten eines Implantates eine stärkere, eine schwächere oder gar keine Röntgensichtbarkeit erwünscht ist, kann der Einsatz und die Einsatzmenge der Fasern aus röntgenopaken Werkstoffen gesteuert werden. Es ist also die Möglichkeit der Konzentration bzw. Anhäufung dieser Fasern von besonderer Bedeutung.

In diesem Zusammenhang ist es dann auch möglich, daß bezogen auf die längs- oder querorientierte Ausrichtung in einem zu fertigenden Bauteil Bereiche unterschiedlicher Faserorientierung oder unterschiedlichen Faserverlaufes vorgesehen sind. Auch auf diese Weise kann eine noch mehr Aufschluß gebende Röntgendiagnostik positiv beeinflußt werden.

Eine besondere Ausfuhrungsvanante sieht dabei vor, daß ein Gesamtfaseranteil von 50 Vol-% vorgesehen ist und das Verhältnis von Kohlenstofffasern zu Fasern oder faserartigen Teilen aus einem Material mit höherer Röntgenabsorption variabel ist. Es wird damit Verbundwerkstoff mit gleichen oder sogar noch besseren Festigkeitswerten erzielt, obwohl der Gesamtvolumanteil der Fasern nicht erhöht wird.

Damit Bauteile aus dem erfindungsgemäßen Verbundwerkstoff exakt den Einsatzbedingungen angepaßt werden können, wird vorgeschlagen, daß über die Länge oder die Breite eines aus dem Verbundwerkstoff zu fertigenden Bauteiles der Gesamtfaseranteil im Verbundwerkstoff gleichbleibend ist, dass aber das Verhältnis von Kohlenstofffasern zu Fasern oder faserartigen Teilen aus einem Material mit höherer Röntgenabsorption variabel ist. Es kann damit die Sichtbarkeit für eine optimale Röntgendiagnostik bewußt gesteuert werden, ohne daß damit eine Beeinträchtigung der Festigkeitswerte stattfinden würde.

Es ist aber im Rahmen der Erfindung auch möglich, daß die Steifigkeit eines aus dem Verbundwerkstoff zu fertigenden Bauteiles durch unterschiedliche Orientierung der Fasern vom Angriffsende zum freien Ende hin variiert. Dies kann bei einem Verbindungselement, also z.B. bei einer Schraube, erwünscht sein, wenn beim Einsatz verschiedene Bereiche eine größere Biegsamkeit aufweisen sollen als andere Abschnitte. Es kann dadurch eine exakte Anpassung auch an die Gegebenheiten im Bereich eines Knochens erfolgen.

Dabei ist es nicht nur möglich, eine stufenlose Einstellung der Festigkeit eines solchen Bauteiles zu erreichen. Es wird auch vorgeschlagen, daß die Steifigkeit eines aus dem Verbundwerkstoff zu fertigenden Bauteiles durch unterschiedliche Orientierung der Fasern vom Angriffsende zum freien Ende hin stufenförmig oder kontinuierlich abnimmt.

Bei einer besonderen Ausführungsvariante ist in einem aus dem Verbund werkstoff gefertigten Bauteil, das als eine Osteosyntheseplatte einsetzbar ist, in einem Bereich von einer oder mehreren Ausnehmungen oder Löchern eine Konzentration von Fasern vorhanden, wobei gegebenenfalls der Anteil von Fasern oder faserartigen Teilen aus einem Material mit höherer Röntgenabsorption in dem Bereich verringert ist. Es kann damit dafür gesorgt werden, daß nicht in einem Bereich mit stark konzentrierter Anordnung von Fasern auch eine starke Konzentration von Fasern aus einem Material mit höherer Röntgenabsorption erfolgt. Dies wäre u.U. für eine zielführende Röntgendiagnostik nicht vorteilhaft. Im Gegensatz dazu kann dadurch erreicht werden, daß der Gehalt an Fasern aus einem Material mit höherer Röntgenabsorption wie gewünscht über die ganze Länge und/oder Breite eines Bauteiles, also auch im Bereich von Ausnehmungen oder Löchern gleichbleibend ist.

Es sind also durch die Verwendung des erfindungsgemäßen Verbundwerkstoffes und durch den Einsatz desselben bei der Herstellung von Bauteilen viele neue Möglichkeiten geschaffen worden, um auch beim Einsatz von Implantaten aus solchen Werkstoffen eine optimale Röntgendiagnostik durchführen zu können.

Weitere Einzelheiten werden in der nachstehenden Beschreibung anhand der Zeichnung noch näher erläutert. Es zeigen:
Fig.1 ein Bauteil in Form einer Knochenschraube;
Fig.2 ein Bauteil in Form einer Osteosyntheseplatte.

Bei der vorliegenden Erfindung geht es einerseits um einen Verbundwerkstoff aus Polymer- oder Keramikmaterial mit einem Gehalt an integrierten Verstärkungselementen in Form von Fasern oder faserartigen Teilen zur Herstellung von auf Zug, Biegung, Scherung, Druck und/oder Torsion beanspruchten Bauteilen für den Einsatz bei Implantaten wie Osteosyntheseplatten, Endoprotheseteilen, Verschraubungselementen, bei chirurgischen Instrumenten od.dgl., und andererseits um aus einem solchen Verbundwerkstoff hergestellte Bauteile, wie sie vorstehend bereits aufgezählt worden sind. Es wird dabei als wesentlich angesehen, in dem Polymer- oder Keramikmaterial zumindest einen geringen Anteil des Gehalts an Fasern oder faserartigen Teilen aus einem Material vorzusehen, dessen Röntgenabsorption höher ist als die der übrigen Fasern oder faserartigen Teile.

Der Verbundwerkstoff besteht bei einem Ausführungsbeispiel aus einem Polymer- oder Keramikmaterial mit einem Faseranteil von mehr als 50 Vol-% mit überwiegendem Einsatz von Endlosfasern. Zumindest ein geringer Anteil von Fasern oder faserartigen Teilen besteht aus einem Material mit höherer Röntgenabsorption als die der übrigen Fasern oder faserartigen Teile. Es kann dabei eine Vorfertigung als Profilstangenmaterial aus Thermoplasten mit Kohlenstoffasern und Fasern aus einem Material mit höherer Röntgenabsorption erfolgen. In einem Warmumformverfahren erfolgt dann die endgültige Herstellung des Bauteiles aus dem Verbundwerkstoff. Der Werkstoff wird also in eine für das endgültige Bauteil erforderliche Form gepreßt. Eine spezielle Variante sieht dabei vor, daß der Verbundwerkstoff aus mit Kohlenstoffasern verstärktem PAEK (Poly-Aryl-Ether-Keton) und einem Anteil von Fasern aus einem Material mit höherer Röntgenabsorption gebildet ist Trotz der Fasern aus einem Material mit höherer Röntgenabsorption bleibt eine optimale Verarbeitungsmöglichkeit erhalten und es entsteht kein zusätzlicher Werkzeugverschleiß. Es ist nicht nur eine Verarbeitbarkeit durch Pressen in einem Warmumformverfahren gegeben, sondern es ist auch eine Fertigung in einem Spritzgußverfahren möglich.

Durch den Einsatz des Verbundwerkstoffes ist auch die Biokompatibilität des fertigen Bauteiles gewährleistet

Die Fasern oder faserartigen Teile aus einem Material mit höherer Röntgenabsorption in dem Verbundwerkstoff sind aus einem nichtmagnetischen Werkstoff gebildet Besonders geeignet sind dabei Fasern oder faserartige Teile mit höherer Röntgenabsorption aus Tantal, Wolfram, Gold, Platin od.dgl., also einem Metall mit hohem Attenuationskoeffizienten. Im Rahmen der Erfindung wäre es auch denkbar, z.B. keramische Fasern aus Oxiden von Elementen mit höherer Röntgenabsorption einzusetzen. Unter faserartigen Teilen können u.a. auch Langfasern oder Kurzfasern oder auch zusätzliche andere Füllstoffe verstanden werden, weiche, ohne die Festigkeit zu verringern, einzusetzen sind. Es ist möglich, zu den vorhandenen Verstärkungselementen gleiche oder gleichartige Verstärkungselemente in Form von Fasern oder faserartigen Teilen einzusetzen. Unter "gleichartig" werden dabei eine gleiche oder gleichartige Dimension und/oder gleiche oder gleichartige mechanische Eigenschaften verstanden.

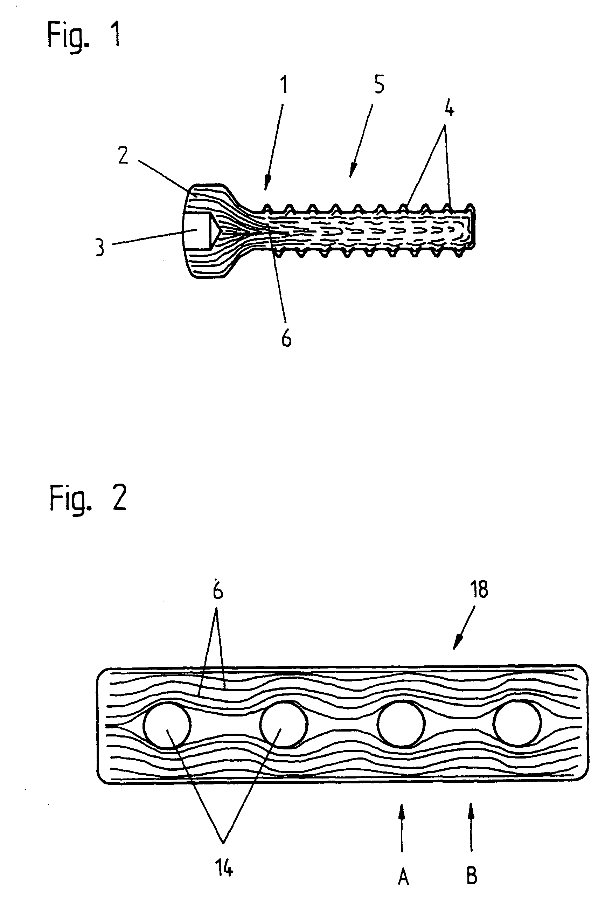

Bei den Darstellungen auf der Zeichnung kann nur in sehr geringem Maße das Wesen der Erfindung aufgezeigt werden. Es bedarf also der zusätzlichen nachstehenden Erläuterungen. Das in Fig.1 dargestellte Bauteil 1 in Form einer Schraube besteht im Wesentlichen aus einem Kopf 2, einem Angriff 3 für die Krafteinleitung von einem Drehwerkzeug her und einem mit einem Gewinde versehenen Schaft 5. Bei einem solchen Bauteil 1 geht es um den besonderen Verlauf und die Anordnung von Endlosfasern 6. Durch die Wahl eines Verbundes von Thermoplasten mit Kohlenstoffasern läßt sich ein leichtes, röntgentransparentes und biokompatibles Verbindungselement schaffen. Um aber gerade bei einer Röntgendiagnostik dieses Verbindungselement sichtbar zu machen, bedarf es der erfindungsgemäßen Maßnahmen, indem nämlich ein Teil der Fasern 6 aus einem Material mit höherer Röntgenabsorption besteht

Die erfindungsgemäßen Maßnahmen lassen sich praktisch bei allen lmplantaten einsetzen, also auch bei schienen- oder plattenartigen Bauteilen 18. In Fig.2 ist schematisch ein solches Bauteil 18 in Form einer Osteosyntheseplatte dargestellt Bei solchen Bauteilen sind Durchgangsöffnungen 14 , Einbuchtungen, Sacklöcher usw. vorgesehen, welche dann in spezieller Weise von den Fasern umgeben sind. Ohne irgendwelche zusätzlichen Maßnahmen bezüglich einer bewußten Steuerung der Menge und der Ausrichtung der Fasern 6 ergibt sich in üblicherweise geschwächten Zonen A eine dichtere Anordnung von Fasern 6, so daß diese Zonen A die gleiche Festigkeit oder Steifigkeit wie in anderen Bereichen B eines solchen Bauteiles haben. Bei einer Herstellung in einem Warmumformverfahren, insbesondere durch ein Gegentaktfließpressen, können der Verlauf und die Ausrichtung der Fasern 6 noch zusätzlich gesteuert und somit beeinflußt werden.

In vorteilhafter Weise sind alle eingesetzten Fasern 6 oder ist zumindest ein großer Anteil derselben, also die Kohlenstofffasern und die Fasern aus einem Material mit höherer Röntgenabsorption, als Endlosfasern oder Fasern mit einer Länge von mehr als 3 mm ausgebildet. Dabei wird aus Festigkeitsgründen darauf geachtet, die eingesetzten Fasern sowohl im Rohling als auch im fertigen Bauteil oberflächendeckend vom Matrixmaterial umschlossen auszuführen.

In dem herzustellenden Bauteil 1 oder 18, also beispielsweise einer Schraube gemäß Fig.1 oder einer Osteosyntheseplatte gemäß Fig.2, aus einem Verbundwerkstoff sind in Anpassung an die Form und den Einsatz des Bauteiles 1 oder 18 ein vorherbestimmbarer Verlauf und eine vorherbestimmbare Menge und Ausrichtung der Verstärkungselementen in Form von Fasern 6 oder faserartigen Teilen aus einem Material mit höherer Röntgenabsorption vorgesehen. Es können auch bezogen auf die längs- oder querorientierte Ausrichtung des Bauteiles 1 oder 18 Bereiche unterschiedlicher Faserorientierung oder unterschiedlichen Faserverlaufes vorgesehen sein.

Bei einem Bauteil 1 oder 18 ist schließlich bei einem Gesamtfaseranteil von beispielsweise 50 Vol-% das Verhältnis von Kohlenstofffasern 6 zu Fasern 6 oder faserartigen Teilen aus einem Material mit höherer Röntgenabsorption je nach Anwendungsanforderung veränderbar oder verändert. Es ist auch möglich, über die Länge oder die Breite eines Bauteiles 1 oder 18 den Gesamtfaseranteil im Verbundwerkstoff gleichbleibend vorzusehen, wobei sich jedoch je nach Bedarf und je nach Anwendungsanforderung das Verhältnis von Kohlenstofffasern 6 zu Fasern 6 oder faserartigen Teilen aus einem Material mit höherer Röntgenabsorption verändert. Es ist auch möglich, die Steifigkeit des Verbindungselementes durch unterschiedliche Orientierung der eingesetzten Fasern vom Angriffsende zum freien Ende hin zu variieren. Eine weitere Variante ist darin zu sehen, wenn die Steifigkeit des Bauteiles durch unterschiedliche Orientierung der Fasern vom Angriffsende her gesehen zum freien Ende hin stufenförmig oder kontinuierlich abnimmt.

Gerade bei einem Bauteil 18 in Form eines streifen- oder plattenförmigen Montageteiles, z.B. einer Osteosyntheseplatte, wie es in Fig.2 dargestellt ist, ist im Bereich A von einer oder mehreren Ausnehmungen 14 oder Löchern eine Konzentration von Fasern 6 vorhanden. Hier ist es möglich, bei Bedarf den Anteil von Fasern 6 oder faserartigen Teilen aus einem Material mit höherer Röntgenabsorption in diesen Bereichen A zu verringern. Wenn hingegen auch im Bereich dieser Konzentration von Fasern der Anteil der Fasern aus einem Material mit höherer Röntgenabsorption nicht verringert wird, können sich bei einem Röntgeneinsatz noch bessere Kontraste beim Zielen ergeben.

## Patentansprüche

1. Verbundwerkstoff aus Polymer- oder Keramikmaterial mit einem Gehalt an integrierten Verstärkungselementen in Form von Fasern oder faserartigen Teilen für auf Zug, Biegung, Scherung, Druck und/oder Torsion beanspruchte Bauteile für den Einsatz bei Implantaten, Osteosyntheseplatten, Endoprotheseteilen, Verschraubungselementen oder bei chirurgischen Instrumenten, **dadurch gekennzeichnet, dass** in dem Polymer- oder Keramikmaterial zumindest ein geringer Anteil der Verstärkungselemente durch Verstärkungselemente ersetzt ist, die aus einem Material bestehen, dessen Röntgenabsorption höher als die des Materials der übrigen Verstärkungselemente ist.

2. Verbundwerkstoff nach Anspruch 1, **dadurch gekennzeichnet, dass** dieser aus einem Polymer- oder Keramikmaterial mit einem hohen Anteil an Endlos-, Lang- oder Kurzfasem besteht.

3. Verbundwerkstoff nach Anspruch 1 oder 2, **dadurch gekennzeichnet, dass** dieser als Profilstangenmaterial aus Thermoplasten mit Kohlenstofffasern und Fasern aus einem Material mit höherer Röntgenabsorption besteht.

4. Verbundwerkstoff nach einem der Ansprüche 1 bis 3, **dadurch gekennzeichnet, dass** dieser aus mit Kohlenstofffasern verstärktem PAEK (Poly-Aryl-Ether-Keton) und einem Anteil von Fasern aus einem Material mit höherer Röntgenabsorption gebildet ist.

5. Verbundwerkstoff nach einem der Ansprüche 1 bis 4, **dadurch gekennzeichnet, dass** die Kohlenstofffasern und die Fasern aus einem Material höherer Röntgenabsorption als Endlosfasern und/oder Fasern mit einer Länge von mehr als 3 mm ausgebildet sind.

6. Verbundwerkstoff nach den Ansprüchen 1 bis 5, **dadurch gekennzeichnet, dass** die eingesetzten Fasern (6) oder faserartigen Teile sowohl im Rohling als auch im fertigen Bauteil (1, 18) oberflächendeckend vom Matrixmaterial umschlossen sind.

7. Verbundwerkstoff nach einem der Ansprüche 1 bis 6, **dadurch gekennzeichnet, dass** die Fasern (6) oder faserartigen Teile aus einem Material mit höherer Röntgenabsorption aus einem nichtmagnetischen Werkstoff gebildet sind.

8. Verbundwerkstoff nach einem der Ansprüche 1 bis 7, **dadurch gekennzeichnet, dass** die Fasern (6) oder faserartigen Teile mit höherer Röntgenabsorption aus Tantal, Wolfram, Gold oder Platin, also aus einem Metall oder aus Metalloxiden mit hohem Attenuationskoeffizienten bestehen.

9. Verbundwerkstoff nach einem der Ansprüche 1 bis 8, **dadurch gekennzeichnet, dass** bezogen auf die längs- oder querorientierte Ausrichtung in einem zu fertigenden Bauteil (1, 18) Bereiche unterschiedlicher Faserorientierung oder unterschiedlichen Faserverlaufes vorgesehen sind.

10. Verbundwerkstoff nach einem der Ansprüche 1 bis 9, **dadurch gekennzeichnet, dass** ein Gesamtfaseranteil von 50 Vol-% vorgesehen ist und das Verhältnis von Kohlenstofffasern zu Fasern oder faserartigen Teilen aus einem Material mit höherer Röntgenabsorption variabel ist.

11. Verbundwerkstoff nach einem der Ansprüche 1 bis 10, **dadurch gekennzeichnet, dass** über die Länge oder die Breite eines aus dem Verbundwerkstoff zu fertigenden Bauteiles der Gesamtfaseranteil in dem Verbundwerkstoff gleichbleibend ist, dass aber das Verhältnis von Kohlenstofffasern (6) zu Fasern (6) oder faserartigen Teilen aus einem Material mit höherer Röntgenabsorption variabel ist.

12. Verbundwerkstoff nach einem der Ansprüche 1 bis 11, **dadurch gekennzeichnet, dass** die Steifigkeit eines aus dem Verbundwerkstoff zu fertigenden Bauteiles durch unterschiedliche Orientierung der eingesetzten Fasern (6) vom Angriffsende zum freien Ende hin variiert.

13. Verbundwerkstoff nach einem der Ansprüche 1 bis 12, **dadurch gekennzeichnet, dass** die Steifigkeit eines aus dem Verbundwerkstoff zu fertigenden Bauteiles (1) durch unterschiedliche Orientierung der Fasern vom Angriffsende zum freien Ende hin stufenförmig oder kontinuierlich abnimmt.

14. Verbundwerkstoff nach einem der Ansprüche 1 bis 13, **dadurch gekennzeichnet, dass** in einem aus dem Verbundwerkstoff gefertigten Bauteil, das als Osteosyntheseplatte einsetzbar ist, in einem Bereich (A) von einer oder mehreren Ausnehmungen (14) oder Löchern eine Konzentration von Fasern (6) vorhanden ist, wobei gegebenenfalls der Anteil von Fasern (6) oder faserartigen Teilen aus einem Material mit höherer Röntgenabsorption in dem Bereich verringert ist.

## Claims

1. A composite material of polymeric or ceramic material with a content of integrated reinforcing elements in the form of fibres or fibrous elements for components subjected to tension, bending, shearing, compression and/or torsion for use in implants, osteosynthesis plates, endoprosthetic parts, screw-connection elements or in surgical instruments, **characterised in that** in the polymeric or ceramic material at least a small proportion of the reinforcing elements is replaced with reinforcing elements which consist of a material whose x-ray absorption is higher than that of the material of the other reinforcing elements.

2. A composite material according to Claim 1, **characterised in that** it consists of a polymeric or ceramic material with a high proportion of endless, long or short fibres.

3. A composite material according to Claim 1 or 2, **characterised in that** as sectional rod material it consists of thermoplastics with carbon fibres and fibres of a material having higher x-ray absorption.

4. A composite material according to any one of Claims 1 to 3, **characterised in that** it consists of PAEK (poly-aryl-ether-ketone) reinforced with carbon fibres and a proportion of fibres of a material having higher x-ray absorption

5. A composite material according to any one of Claims 1 to 4, **characterised in that** the carbon fibres and the fibres of a material of higher x-ray absorption are in the form of endless fibres and/or fibres of a length of more than 3 mm.

6. A composite material according to Claims 1 to 5, **characterised in that** the fibres (6) or fibrous elements used are surrounded both in the blank and in the finished component (1,18) by matrix material covering the surface.

7. A composite material according to any one of Claims 1 to 6, **characterised in that** the fibres (6) or fibrous elements of a material with higher x-ray absorption are formed from a non-magnetic material.

8. A composite material according to any one of Claims 1 to 7, **characterised in that** the fibres (6) or fibrous elements with higher x-ray absorption consist of tantalum, tungsten, gold or platinum, i.e. of a metal or metal oxides with a high attenuation coefficient.

9. A composite material according to any one of Claims 1 to 8, **characterised in that**, in relation to the longitudinally or transversely oriented alignment in a component (1,18) to be produced, zones of different fibre orientation or different fibre direction are provided.

10. A composite material according to any one of Claims 1 to 9, **characterised in that** a total fibre content of 50 % by volume is provided and ratio of carbon fibres to fibres or fibrous elements of a material with higher x-ray absorption is variable.

11. A composite material according to any one of Claims 1 to 10, **characterised in that** the total fibre content in the composite material is constant over the length or the width of a component to be produced from the composite material, but **in that** the ratio of carbon fibres (6) to fibres (6) or fibrous elements of a material with higher x-ray absorption is variable.

12. A composite material according to any one of Claims 1 to 11, **characterised in that** the rigidity of a component to be produced from the composite material varies from the engagement end towards the free end as a result of differing orientation of the fibres (6) used.

13. A composite material according to any one of Claims 1 to 12, **characterised in that** the rigidity of a component (1) to be produced from the composite material decreases in stages or continuously from the engagement end towards the free end as a result of differing orientation of the fibres.

14. A composite material according to any one of Claims 1 to 13, **characterised in that** in a component produced from the composite material, which can be used as an osteosynthesis plate, a concentration of fibres (6) is provided in a zone (A) of one or more recesses (14) or holes, wherein optionally the proportion of fibres (6) or fibrous elements of a material with higher x-ray absorption is reduced in this zone.

## Revendications

1. Matériau composite constitué de matériau polymère ou céramique ayant une teneur en éléments de renforcement intégrés sous forme de fibres ou de parties de type fibres pour composants sollicités par la traction, la flexion, le cisaillement, la pression et/ou la torsion pour l'utilisation dans les implants, les plaques d'ostéosynthèse, les pièces d'endoprothèse, les éléments de vissage ou dans les instruments chirurgicaux,
**caractérisé en ce que**
dans le matériau polymère ou céramique, au moins une faible proportion des éléments de renforcement est remplacée par des éléments de renforcement en un matériau dont l'absorption des rayons X est supérieure à celle du matériau des autres éléments de renforcement.

2. Matériau composite selon la revendication 1,
**caractérisé en ce qu'**
il est constitué d'un matériau polymère ou céramique comportant une proportion élevée de fibres continues, longues ou courtes.

3. Matériau composite selon la revendication 1 ou 2,
**caractérisé en ce qu'**
il est constitué en tant que matériau en tiges profilées, en matériaux thermoplastiques avec fibres de carbone, et fibres en un matériau ayant une absorption plus élevée des rayons X.

4. Matériau composite selon l'une des revendications 1 à 3,
**caractérisé en ce qu'**
il est formé de polyaryléther cétone renforcée par des fibres de carbone et d'une proportion de fibres en un matériau ayant une absorption plus élevée des rayons X.

5. Matériau composite selon l'une des revendications 1 à 4,
**caractérisé en ce que**
les fibres de carbone et les fibres en un matériau ayant une absorption plus élevée des rayons X sont des fibres continues et/ou des fibres ayant une longueur supérieure à 3 mm.

6. Matériau composite selon l'une des revendications 1 à 5,
**caractérisé en ce que**
les fibres (6) ou les parties de type fibres utilisées sont entourées sur toute leur surface par un matériau formant une matrice, aussi bien dans l'ébauche que dans le composant fini (1, 18).

7. Matériau composite selon l'une des revendications 1 à 6,
**caractérisé en ce que**
les fibres (6) ou les parties de type fibres en un matériau ayant une absorption plus élevée des rayons X, sont constituées d'un matériau non magnétique.

8. Matériau composite selon l'une des revendications 1 à 7,
**caractérisé en ce que**
les fibres (6) ou les parties de type fibres ayant une absorption plus élevée des rayons X sont constituées de tantale, de tungstène, d'or ou de platine, également d'un métal ou d'oxydes métalliques ayant un coefficient d'atténuation élevé.

9. Matériau composite selon l'une des revendications 1 à 8,
**caractérisé en ce que**
des zones d'orientation différente des fibres ou de disposition différente des fibres, par rapport à la direction longitudinale ou transversale, sont prévues dans un composant à fabriquer (1, 18).

10. Matériau composite selon l'une des revendications 1 à 9,
**caractérisé en ce qu'**
une proportion totale de fibres de 50 % en volume est prévue et le rapport des fibres de carbone aux fibres ou parties de type fibres en un matériau ayant une absorption plus élevée des rayons X est variable.

11. Matériau composite selon l'une des revendications 1 à 10,
**caractérisé en ce que**
la proportion totale de fibres dans le matériau composite est constante sur la longueur ou la largeur d'un composant à fabriquer dans le matériau composite, mais le rapport des fibres de carbone (6) aux fibres (6) ou parties de type fibres en un matériau ayant une absorption plus élevée des rayons X est variable.

12. Matériau composite selon l'une des revendications 1 à 11,
**caractérisé en ce que**
la rigidité d'un composant à fabriquer dans le matériau composite varie depuis l'extrémité de prise jusqu'à l'extrémité libre en raison de la différence d'orientation des fibres utilisées (6).

13. Matériau composite selon l'une des revendications 1 à 12,
**caractérisé en ce que**
la rigidité d'un composant (1) à fabriquer dans le matériau composite diminue par paliers ou en continu depuis l'extrémité de prise jusqu'à l'extrémité libre en raison de la différence d'orientation des fibres.

14. Matériau composite selon l'une des revendications 1 à 13,
**caractérisé en ce que**
dans un composant fabriqué dans le matériau composite et utilisé comme plaque d'ostéosynthèse, il existe une concentration de fibres dans une zone (A) d'un ou de plusieurs évidements (14) ou trous et, le cas échéant, la proportion de fibres (6) ou de parties de type fibres en un matériau ayant une absorption plus élevée des rayons X est réduite dans cette zone.
